# EUROPEAN PATENT APPLICATION

(11) **EP 3 340 193 A1**
(43) Date of publication of application: **27.06.2018**
(21) Application number: 16839692.7
(22) Date of filing: 16.08.2016
(51) Int. Cl.: G07F 11/00

(54) **VENDING MACHINE FOR DISTANCE SELLING REGULATED GOODS**

(30) Priority: 21.08.2015 RU 2015135449
(71) Applicant: Pishchik, Kirill Eduardovich, Moscow 119034 (RU)
(72) Inventor: Pishchik, Kirill Eduardovich, Moscow 119034 (RU)
(74) Representative: Petosevic, Slobodan
(86) International application number: PCT/RU2016/000552
(87) International publication number: WO 2017/034441

(57) **Abstract**

Present invention refers to the devices and methods for automated selling, and particularly relates to vending machine for remote selling of goods purchase of which requires authorization or is subject to age restrictions.

Proposed a remotely controlled vending machine for selling of regulated goods including hardware and software unit of control commands receipt and interpretation by the vending machine and the related functional features and modules of the vending machine for acceptance, payment and dispensing of the order, equipped with video control means and controlled remotely; the vending machine is also equipped with goods return and payment modules if there is the need, as well as the communication unit for the vending machine connection to station of operators-specialists. Besides the vending machine is equipped with the means making possible its usage by the weak-eyed people by means of identification of the means of communication with the operator-specialist, payment and goods dispensing using the graphic indicators in the form of Braille type.

Method for selling of the regulated goods is implemented using the proposed vending machine for remote selling providing control and management of the ordering stage, checking of the authorization documents, payment, dispensing and, if required, return of the goods by the operator-specialist. The method provides for communication and remote control of the vending machine and the selling process of at least one vending machine controlled by the operator-specialist from computerized workstation of operators-specialists.

Proposed vending machine and method for remote selling of the regulated goods makes it possible to provide secure and convenient process of the regulated goods selling to the authorized persons as well as to provide availability of the vending machine for the handicapped people, including, the weak-eyed people.

## Description

### Field of the invention:

This invention refers to devices and methods for automated remote selling, and particularly relates to vending machines for selling of goods, purchase of which requires authorization or is subject to age restrictions.

Vending machines became widespread for selling of various piece goods, which are normally related to customer-size goods and have obvious advantage over sales outlets and premises in terms of accessibility, low-occupied area and low maintenance costs. Vending machines are also used for sales of regulated goods selling of which is subject to age restrictions, for example tobacco and alcohol, including medicines. Vending machines selling the prescription medicines have become more and more widespread, which entails the need for the vending machines equipped with active means of sales control and operator's participation.

As is known, there is a machine selling goods that require personal identification of the purchaser, see patent of the Russian Federation for utility model RU73106,U1 [1] published on 10.05.2008, consisting of a body, a show-case with the goods specimens (ordering cell), a device for storage and dispensing of goods (order dispensing module), a data display device in the form of monitor with active screen (touch-screen), payment tool in the form of cash receiving device (cash receiving module) in particular, a cash acceptor with the function of change-making and receipt printer (receipt-printing and change-making module). If required, the vending machine may be equipped with scanning device for scanning the identity document and means of video-telephone communication with operator.

Selling method with usage of such vending machine performs selling of one or several types of goods with execution of all selling operations by the vending machine itself. The method also includes the option of interruption of the selling process or selling of the goods requiring identification of the purchaser for contacting the Call-center operator where the purchaser's facial image is transmitted via webcam and for identification by means of scanning device deciding whether further operations may be performed by the vending machines following the results of visual check of the purchaser's age for compliance with age restrictions.

Similar solution is proposed in application US2009/0276088 [2] published on 5.11.2009. The solution relates to vending machine and method for selling of regulated goods, sales of which is subject to age restrictions and includes unlocking of access to the vending machine functions after visual check of the purchaser's documents by the operator.

The mentioned vending machines [1, 2] and methods for selling of regulated goods essentially provide for only controlled unlocking of access to the vending machine following the results of the purchaser's age identification and further actions of the purchaser performed without participation of Call-center operator. However, in order to ensure warranted selling of the controlled goods as intended the whole selling process from the moment of the purchase beginning till its completion shall be controlled with obligatory participation of operator but not by the vending machine itself as regards the sales of the goods requiring submission of the authorization document, for example, medical prescription via the vending machine.

There is a method for selling of medicines including prescription medicines via the vending machine where the purchaser inserts the prescription into the reading device and then pays for the purchase using the cash insertion slot (cash acceptor or coin acceptor) or by means of credit card using the card insertion slot; see electronic resource of the Internet URL: http:www.1000ideas.ru/article/biznes/moda-krasotazdorove/biznes-ideya-1886-avtomat-dlya-prodazhi-tabletok/ [3] as example. After the payment, the purchaser gets the receipt and medication regimen printout. During the purchase of medicine, the purchaser may contact the peddler that will check the medical prescription and provide consultation on medication regimen. The method is implemented in the vending machine containing the body, medical prescription acceptor, payment module acceptor, goods dispensing tray, receipt and medication regimen printing module as well as means of audio/video communication with peddler. All operations from the beginning of purchasing process until its termination are performed by the vending machine without participation of operator.

Solving the problem of the regulated goods sales, the vending machine does not ensure sufficient security in terms of warranted selling of the prescription medicines as intended in view of absence of the means providing for sequential checking and control of the goods purchase from the moment of the goods selection until its dispensing. Functional capabilities of such vending machine are limited only by sales of goods with no provisions regarding the return of goods in case of the purchaser's erroneous actions and, consequently, return of money.

Besides, vending machines in the related art have limited usability for certain categories of handicapped people, in particular, for weak-eyed people and does not ensure warranted identification of the purchaser's identity, which increases the risk of selling the prescription medicine other than intended. In addition, the medicines vending machines in the related art do not provide the purchaser with convenient access to certificate of the medicine, if required, since reading the certificate from the screen is cumbersome and is almost impossible for the weak-eyed people.

Purpose of present invention consists in development of a complex on remote sales of regulated goods with participation of operator-specialist on the regulated goods as well as the vending machine and the method for selling of regulated goods, sales of which is subject to relevant legislative acts, in particular, for selling of prescription medicines in compliance with the basic provisions of the Law on medicines selling by means of sales process controlled by operator-pharmacist or operator-peddler.

Proposed remotely-controlled vending machine and method for distance selling of the controlled goods make it possible to attain the technical result consisting in provision of secure and convenient process of sales of regulated goods to authorized people as well as to provide accessibility of the vending machine for the handicapped people, in particular to the weak-eyed people, by means of continuous control of the sales process by operator-pharmacist or operator-peddler with option of direct familiarization with the medicines certificates.

Usage of the vending machines for selling of medicines makes it possible to reduce the price of the medicines for the population by means of decrease of the premises renting costs and salary costs, makes the medicines available for the population from geographic point of view by means of extensive network of pharmaceutical vending machines for the medicines selling. Due to mobility and small occupied area the pharmaceutical vending machines may be located on the tour circuits, fuel filling stations which improves availability of the medicines for people using the motor cars.

The specified technical result is attained by means of remotely controlled vending machines containing
Vending machine for remote selling of regulated goods consisting of the body and the following items installed in the body
communication unit providing the possibility of establishing of communication with remote station of operators-specialists, herewith, the station of operators-specialists includes at least one computerized workstation of operator-specialist on regulated goods, software and hardware unit for data collection and control of operations of the vending machine for remote selling of regulated goods and database and data storage management server.
software and hardware unit of the control commands receipt and interpretation by the vending machine and related vending machine functional features and modules for acceptance, payment and dispensing of the order including
audio and video unit containing the display device in the form of touch screen with purchaser interface installed on the front panel of the body, video camera located over the touch screen, loudspeakers and microphone and
operator-specialist call button equipped with the plate with graphic pointer of designation in the form of Braille type located on the front panel and
controlled by the operator-specialist
module with the cells for goods performed as containers with doors on the front panel with monitor camera of the doors,
authorization document reading device in the form of scanner, at least one payment module with payment acceptor on the front panel of the body,
goods dispensing module with tray on the front panel of the body equipped with monitor camera of dispensing controlled by shutter door,
change and receipt dispensing module with tray on the front panel of the body equipped with monitor camera, and
goods return module on the side panel of the body and documentation storage module with tray on the side panel of the body equipped, accordingly, with controlled shutter doors and monitor cameras of goods return and documentation dispensing, herewith
plates with graphic indicators of designation in the form of Braille type are placed near the points of location of the mentioned payment acceptor, tray of change and receipt dispensing module, tray of goods dispensing module and tray of goods return module.

Workstation of the operator-specialist is the workstation of operator-pharmacist or operator peddler.

The module with cells for goods is preferably equipped with the device keeping the temperature and humidity conditions of the container.

Means of payment for the goods may be performed in the form of cash payment module and/or credit card payment module.

If required, the authorization document reading device may be equipped with container with monitor camera for acceptance of authorization documents, for example prescription forms in case of selling of goods requiring the authorization documents.

Method for remote selling of regulated goods may be implemented using the vending machine for remote selling according to claim 1, herewith, the method includes
connection of one or more vending machines for remote selling of regulated goods to station of operators-specialists by means of wire line or wireless link including at least one computerized workstation of operator-specialist on regulated goods, software and hardware unit for data collection and control of operations of the vending machine for remote selling of regulated goods and database and data storage management server.
initialization of the vending machine from the standby mode by means of touching the touch screen by the purchaser with displaying of the purchaser interface on the screen or by means of pressing the call button on the front panel of the vending machine.
establishing of communication between the vending machine and the station of operators-specialists by the purchaser via wire or wireless communication line by means of touching the icon "call" on the purchaser interface or pressing the call button on the front panel of the vending machine,
determining of available operator-specialist and upload of data on activated vending machine to computerized workstation of operator-specialist, displaying of operation interface of control of the vending machine functional devices and modules on the display of operator-specialist's workstation and initiation of operation mode of the vending machine audio and video unit by the operator-specialist,
displaying of the list of goods requested by the purchaser for acquisition with submission of authorization documents for specific types of goods using the vending machine scanner on the vending machine touch screen with further displaying of the list of selected goods and the goods prices on the screen at the operator-specialist's workstation,
displaying of the purchase total on the vending machine touch screen or in the form of operator-specialist's voice message regarding the goods quantity and price,
confirmation of the selected goods by the purchaser by means of touching the selected goods confirmation icon on purchaser interface or by means of pressing the call button on the vending machine front panel.
unlocking of the vending machine payment module after specification of the customer's payment option (cash/credit card)
payment for the selected goods by means of the payment module acceptor with further unlocking of goods dispensing module and change and receipt dispensing module controlled by operator-specialist using the monitor cameras of the trays of goods dispensing module and change and receipt dispensing module,
putting the vending machine into standby mode by the operator-specialist or automatically upon expiration of time interval during which the vending machine is not used by the purchasers.

During selection of the goods, the operator upon purchaser's request may provide him/her access to the goods documentation by means of tray of the documentation storage module with its further return controlled by the operator-specialist using the monitor camera of the said tray.

The method also provides for the possibility of the goods return by means of the goods return tray controlled by the operator-specialist using the monitor camera of the goods return tray.

Proposed vending machine for remote selling also provides for the method for selling of regulated goods upon advance order, including
receipt of message regarding the vending machine address and number and the time of the ordered goods delivery to the vending machine and the unique purchase code by the purchaser,
initialization of the vending machine from the standby mode by means of touching the touch screen by the purchaser with displaying of the purchaser interface on the screen or by means of pressing the call button on the front panel of the vending machine.
establishing of communication between the vending machine and the station of operators-specialists by the purchaser via wire or wireless communication unit by means of touching the icon "call" on the purchaser interface or pressing the call button on the front panel of the vending machine,
entering of the unique code via the purchaser interface on the vending machine screen or entering of the unique code by operator-specialist on basis of the purchaser's voice message sent via the microphone of the vending machine audio and video unit,
displaying of the purchase total on the vending machine touch screen or in the form of operator-specialist's voice message regarding the goods quantity and price,
confirmation of the order by the purchaser by means of touching the order confirmation icon on purchaser interface or by means of pressing the call button on the vending machine front panel.
reporting of the container number of module with the cells for goods by the operator-specialist via the purchaser interface or by means of voice message
payment by the purchaser for the order by means of the selected payment modules acceptor with further unlocking of the container door of the cells for goods module and change and receipt dispensing module controlled by operator-specialist using the monitor camera of the tray of change and receipt dispensing module,
getting of the medicine from the appropriate container controlled by the operator-specialist using the monitor camera 17 of doors 9 of the said containers,
putting the vending machine into standby mode by the operator-specialist or automatically upon expiration of time interval during which the vending machine is not used by the purchasers.

The proposed invention is explained in the drawings wherein:
Fig. 1 represents general view of the remotely controlled vending machine for remote selling of the regulated goods according to the invention.
Fig. 2 represents the flow-chart explaining the method for remote selling of regulated goods using the vending machine for remote selling.

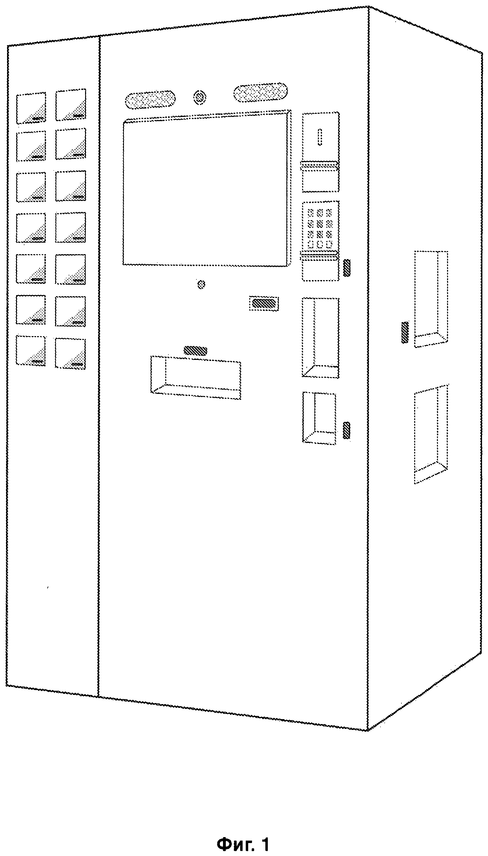

Fig. 1 is a schematic representation of general view of the remotely controlled vending machine for remote sales of the regulated goods.

The vending machine contains the body 3 where devices and functional modules providing placement, storage and selling of the goods are located. The body is performed as supporting frame enclosed with front and side panels and back panel, as a rule, performed in the form of door for provision of access to the devices and functional modules of the vending machine. The body also contains the communication unit intended for communication with remote station of operators-specialists, hardware and software unit providing receipt and interpretation of control commands generated, for example, by operator-specialist on sales of regulated goods.

The vending machine is equipped with audio and video system including display device in the form of touch-screen 4, video camera 5, loudspeaker devices in the form of dynamic speakers 6a and 6b and microphone 7. Video camera 5 is installed with the possibility of capturing both the image of the purchaser area and the image of documents and other text materials which may be submitted by weak-eyed people.

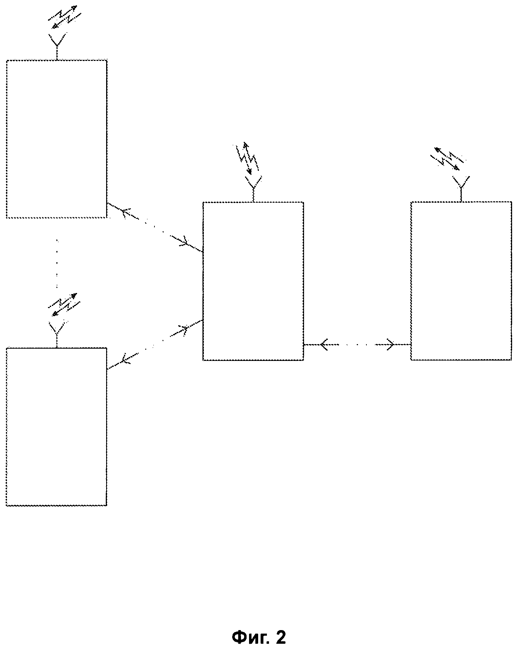

Dynamic speakers are located in such a way as to provide noise-free listening of voice messages, for example, over the data display device. Video camera 5 is located in such a way as to provide the possible broadest capture of the purchaser area, for example, it may be located in the middle over the touch screen 4 between the loudspeakers as specified in fig. 2. Microphone 7 and operator-specialist call button 8 on the surface of which the plate with graphic indicators of designation in the form of Braille type is mounted for informing of the weak-eyed people about designation of the button is located under the touch screen 4. The touch screen is located at eye level determined on basis of data on average height of people.

Audio and video system is used for videoconferences between the purchaser and operator-specialist, consultations during the goods purchasing process as well as during the process of the goods requesting by the purchaser. Besides, the display device and loudspeaker devices are used for familiarization with the goods sold via the vending machine. For example, the purchaser may browse the leaflets to various goods in the booklet mode. In particular, during the sales of medicines the purchaser may get familiarized with the goods description and instruction for use, for example, with regard to a medicine, may browse and listen to advertising of various goods which may be demonstrated when the vending machine is in standby mode.

In order to execute the process of ordering, payment and dispensing of the goods the vending machine is equipped with the modules with goods sales performed as containers for consumer size goods, for example, medicines, authorization document reading device, at least one payment accepting module, in particular, cash accepting module and/or credit card accepting module, change and receipt dispensing module and paid goods dispensing module.

Module with cells for consumer size goods offered for sale is located inside the body (is not visible). The module cells are performed in the forms of containers which may be filled both with the goods of standard range and with the goods outside the standard range. In case the goods outside the standard range are offered, access to the goods is provided for through the door 9 of the container located on the front panel of the vending machine to the left of the touch screen 4. The doors 9 are numbered and contain the plates 9a with the containers numbers marked with graphic indicators of designation in the form of Braille type for the weak-eyed people.

Containers of the saleable goods module are equipped with the means keeping the temperature and humidity conditions of the goods storage. For example, the container of the advance ordered goods module of pharmaceutical vending machine is filled either with medicines outside the standard range of the pharmaceutical vending machine which have been earlier ordered by the purchaser, for instance, via the company's web-site or by means of advance order via the similar pharmaceutical vending machine or with the medicines manufactured by remote centralized prescription department as well by means of advance order.

Work with the payment modules is performed by means of acceptor 10 of the cash (coins or banknotes) accepting module or by means of acceptor 11 of credit card module which are located to the right of the touch screen 4 one under the other.

Window 12 of scanner for input and reading of the authorization document or identity document of the purchaser is located under the credit cards acceptor. Container intended for acceptance of authorization documents which are not to be returned, for example, prescription blanks, equipped with monitor camera (not shown) of the authorization document, for example, medical prescription, input into the container is provided for in the box near the scanner. Tray 13 of change and receipt dispensing module equipped with video camera (not shown) used by operator-specialist for control of change receipt or return of money is located below the scanner. Window 13 is used for return of money in case of erroneous dispensing of the goods, for example, a medicine.

Tray 14 of the goods dispensing module is located in the middle area of the front panel for dispensing of the goods in accordance with the order. Goods dispensing module is used during the purchase. Tray 14 of the module is equipped with shutter door 14b controlled by operator-specialist and monitor video camera (not shown), image from which is transmitted to the operator-specialist's screen during the goods dispensing. Therefore, operator-specialist controls dispensing of those particular goods, for example, medicines which have been ordered and paid by the customer.

Window 12 and trays 13,14 are located at the level convenient for manual actions during scanning of the documents, withdrawal of goods, receipt and change. Tray 13 is also used for return of money in case of erroneous dispensing of the goods, for example, a medicine.

Plates with graphic indicators 10a, 11a, 13a and 14a of designation in the form of Braille type are located near the cash/credit card acceptor, goods dispensing tray and receipt and change dispensing window for convenience of the vending machine usage by the weak-eyed people for payment and receipt of goods and for withdrawal of change and receipt respectively.

Tray 15 of goods return module with lockable shutter door 15b is located on the right side panel of the vending machine body, near to which the plate with graphic indicator 15a of designation in the form of Braille type and tray 16 of the documentation storage module with lockable shutter door 16b are located. Tray 15 of the goods return module and tray 16 of the documentation storage module are controlled by the specialist. Shutter doors 15b and 16b are remotely controlled by operator-specialist. Tray of the goods return module is used in case of erroneous dispensing of the goods, for example, medicines, due to mistake of operator-specialist or employee of logistics service when filling the erroneous cell of the storage module with the goods. Documentation storage module is used for storage of copies of the certificates for the goods, for example, medicines, located in the vending machine. Shutter door 16b of the tray 16 is remotely controlled by the operator-specialist upon the purchaser's request and is controlled by video camera.

Lighting lamps required for illumination of inscriptions in the nighttime, trays and button bar of the vending machine modules may be installed on the vending machine body.

Lockout switches breaking off the circuit when opening the access door to devices and functional module of the vending machine are provided for service staff protection against electric shock.

Each vending machine is also equipped with alarm reporting to the desk of the nearest police department about the attempt of break-in in case if any such attempt and connects all video cameras of the vending machine and starts recording the image of the events taking place inside and outside the vending machine with simultaneous activation of audible alert (siren). Besides, the vending machine is connected to any available operator-specialist who makes additional decisions on resolution of the situation observing the situation in the real time. After that, the decision on the need of sending of the repair crew on duty to the vending machine for direct work with the vending machine is made.

Besides, operator-specialist with additional access may connect to any vending machine and check its characteristics, goods stock and test its proper functionality.

In case of breakdown of the unit, regulating the temperature and humidity conditions of the cells there is another alarm sensitive to the temperature and reporting the failure through the station of operators-specialists.

Proposed method of remote selling with usage of the above described remotely controlled vending machine according to the invention is represented applicable to remote selling of the medicines and the vending machine will be hereinafter referred to as the pharmaceutical vending machine.

Method of remote selling of regulated goods using the vending machine for remote selling of the regulated goods provides for control of one or more vending machine. As explained by the flow chart in fig. 2 one or more vending machines for remote selling of controlled goods are connected to station (2) of operators-specialists by means of wire or wireless communication unit.

Cable lines with usage of cable based on twisted pair of copper conductors (twisted pair), coaxial cable with copper conductor as well as fiber optic cable may be used as wire communication line. Fixed wireless communication on basis of multipoint wireless communication channel carrying out data transmission in radio frequency band may be used as wireless communication network. Each vending machine and station of operators-specialists are equipped with transmitting and receiving antenna and necessary equipment for data preparation and transmission.

Vending machine is equipped with hardware and software unit providing receipt and interpretation of control commands of the vending machines functional features and modules, preparation of data on vending machine for transmission to station of operators-specialists, interpretation of control signals from station of operators-specialists.

Station of operators-specialists is equipped with computerized workstations, hardware and software unit with databases and data storage control servers. Hardware and software unit of the station of operators-specialists provides for the process of data collection and preparation and transmission of operations control signals of the vending machine for remote selling of regulated goods. When the vending machines are used as pharmaceutical vending machines, operators-specialists are pharmacists and peddlers.

A purchaser reaches the pharmaceutical vending machine which at the moment may be in the mode of advertising playback on the touch screen 4 or in the standby mode and performs the vending machine initialization from the advertising playback mode or standby mode by touching the touch screen after which the purchaser interface with "Call" icon will appear. Weak-eyed people put the vending machine into ready mode by pressing the call button 8 on the front panel of the pharmaceutical vending machine equipped with graphic indicator of the "Call" button designation in the form of Braille type.

After pressing of "call" icon on the touch screen 4 or "Call" button 8 the pharmaceutical vending machine 1 is connected to station 2 of operators-specialists. In case of incoming call, the hardware and software unit of the station of operators-specialists connects the pharmaceutical vending machine to computerized workstation of the first available operator-specialist. Herewith the time period to connection to the operator-specialist will be first displayed on the touch screen 4 of the pharmaceutical vending machine or if the operator-specialist is available, video image of the operator-specialist will appear at once and the image of the purchaser, obtained from video camera 5 will appear on the screen of the operator-specialist's workstation, herewith, voice communication is connected as well and so dialog between the operator-specialist and the purchaser begins. Conversation of the operator-specialist and the purchaser is recorded to data storage server of the station of operators-specialists and is stored there for a while for the case of possible investigations in case of non-standard or conflict situations with the purchaser as well as visual recording of the purchaser takes place.

After the operator-specialist's computer is connected to pharmaceutical vending machine operation interface with all control functions of various functional modules and devices of the pharmaceutical vending machine (scanner, payment modules, video cameras, locking devices of shutter door of documentation storage tray 16 and shutter door of goods return module tray 15) will appear on the screen of the operator-specialist's workstation. In addition, the software loads the data on the connected pharmaceutical vending machine, i.e. its geographical position, quantity and names of the medicines contained in the vending machine (stock software) as well as physical parameters (temperature and humidity in various sections of the vending machine). After that, the operator-specialist puts the audio and video unit of the vending machine into operation mode and displays the list of medicines on the touch screen with specification of its price.

During conversation with the operator-specialist the purchaser names the desired goods (for example, medicines) and the operator-specialist selects the medicines requested by the purchaser from the warehouse database and the selection results are displayed on the touch screen 4 of the vending machine in the form of list with specification of the medicines names, its quantity and prices as well as "Total" sums. If necessary, upon the purchaser's request operator-specialist gives consultation on the medicines and the ordering procedure using the audio and video unit of the pharmaceutical vending machine. In case submission of authorization document, for example, medical prescription, is required, the operator-specialist unlocks the scanner and the purchaser scans the medical prescription. After the order is completed, on the purchaser interface of the pharmaceutical vending machine icon "Confirm order" will be activated. The purchaser confirms the order by pressing the icon "Conform order" or by pressing the "Call" button 8 of the vending machine and the message on the order confirmation will appear on the screen of the operator-specialist's computer. After that, the operator-specialist activates operation of cash and credit cards accepting modules and the purchaser may use one of them using acceptor 10 or acceptor 11.

In case the pharmaceutical vending machine is used by the weak-eyed people, the process of medicines selection is carried out using the voice dialog mode of conversation between the purchaser and the operator-specialist. Operator-specialist informs the purchaser about available medicines and generates the list of selected medicines and informs the purchaser with voice about the selection total and price total. Order confirmation is carried out by the purchaser by pressing the "Call" button 8. In case the payment is performed using the credit card, plate 11a with graphic indicator of designation in the form of Braille type is located near credit card slot helping to find the slot. In cays the purchaser pays in cash, he/she uses the cash acceptor (10), near to which one may also find the plate with graphic indicator of designation in the form of Braille type (10a). In both cases operator-specialist controlling all the actions performed by the weak-eyed purchaser by means of video communication helps him to find the modules.

As soon as the required amount of money is inserted or the transaction on the credit card account is completed which is displayed on the operator-specialist's screen, settlement with the purchaser takes place upon the operator-specialist's command: the goods, for example, medicines, are dispensed and the change is made, if necessary. Goods dispensing and change making process is activated and controlled by the operator-specialist using the video cameras of goods dispensing tray 14 and change and receipt dispensing tray 13. The weak-eyed people find the trays using the plates with graphic indicators 14a and 13a of trays designation in the form of Braille type with participation of operator-specialist if necessary.

When more than one medicine is ordered, the pharmaceutical vending machine dispenses the medicines one after another, herewith the sequence is from the larger quantity to the lesser, which simplifies visual control of the medicines dispensing by operator-specialist, since the packages of medicines getting in the tray from the top to the less extent cover the bottom packages. After that, change and receipt are dispensed to the change dispensing tray.

In case the purchaser need a prescription medicine, operator-specialist asks the purchaser to submit the medical prescription. Dispensing of the prescription medicine is as follows: the specialist asks the purchaser to insert the prescription form into the scanner, scanned image appears on the specialist's screen and after the specialist has made sure the prescription is correct and valid as well as in case the medicine is available the process runs as purchase of non-prescription medicine and the prescription gets into the container with prescription forms which is controlled by the specialist by means of video camera located in the container. In case the medicine is unavailable in the pharmaceutical vending machine and there are no analogues or the analogues are not suitable for the purchaser, the prescription form is returned to the purchaser from the scanner.

In case the purchaser wants to browse the documentation for medicines located in the pharmaceutical vending machine, the specialist unlocks the shutter door 16b of the tray 16 of the documentation storage module and the purchaser gets the possibility to open the shutter door and take the folder with copies of the certificates for medicines placed in alphabetical order for convenience of search. Return of the folder is controlled by means of video camera located in the tray. After that, the operator-specialist locks the door of the tray or in case of automatic lock the operator-specialist controls closing and locking of the shutter door.

In case when due to technical mistake an erroneous medicine has been dispensed to the purchaser, which is controlled by the operator-specialist using the video camera of goods dispensing tray 14, the operator-specialist asks the purchaser to put the erroneously dispensed medicine into the goods return tray 15. The procedure is as follows: operator-specialist unlocks the shutter door 15b of the goods return tray, the purchaser opens the shutter door and puts there the goods, for example, the medicine package. After the shutter door of the tray is opened video camera located in tray 15 is activated automatically and the specialist controls return of the medicine package and its integrity. Having made sure the goods are returned, the operator-specialist checks that the goods are in the returned goods tray 15 after closing of the shutter door 15b of the tray. After that, the purchaser is either given the required medicine or the money is returned by means of receipt and change dispensing tray 13 located on the front panel of the pharmaceutical vending machine.

After the purchasing process is completed, the vending machine is put into standby mode by the operator-specialist or automatically upon expiration of time interval during which the vending machine is not used by the purchasers.

Proposed invention also provides for selling of regulated goods for which advance order has been performed via the vending machine for remote selling according to present invention. Such selling is carried out in relation to remote selling of the goods outside the standard list of goods sold via the vending machine. For example, it may be related to selling of the medicines manufactured in central prescription department upon advance order.

Containers of the module with cells for goods where the goods ordered in advance are located are used for implementation of such sales. Access to the goods in such containers is carried out by means of opening the door 9 of the appropriate container, number of which is reported to the purchaser after the order is placed in the container. The door is equipped with electronic locking device controlled by operator-specialist when dispensing the goods after payment. Necessary for storage temperature and humidity are kept in the containers by means of the device keeping temperature and humidity conditions.

Selling of the goods dispensed using the containers of goods module is carried out as follows.

In case the purchaser needs the medicine outside the standard list, he/she may execute an order either by means of visiting the company's website and afterward selection of the withdrawal point (address of pharmaceutical vending machine) or order the goods via the vending machine communicating with operator-specialist. In the latter case, the purchaser calls the operator-specialist and notifies him about the goods to be ordered. If required the purchaser submits the authorization document, for example, in the form of scanned image of the document.

After the order is prepared the purchaser is reported the unique purchase code, address of the vending machine via e-mail or SMS-message.

After that, the purchaser having reached the vending machine initializes it by touching the touch screen 6 or pressing the call button 8, herewith, the purchaser interface is displayed on the touch screen. Touching the icon "call" on the purchaser interface the purchaser establishes communication with the station of operators-specialists via the vending machine communication unit and enters the unique purchase code through the purchaser interface. In case of weak-eyed people, the communication with the station of operators-specialists is established by means of repeated pressing of call button 8 and the unique purchase code is entered by operator-specialist by means of voice message of the purchaser sent via the microphone 7 of the vending machine audio and video unit. The operator-specialist displays the data on quantity and price of the purchase as well as number of container with the cells for goods on the screen 4 of the vending machine and informs the purchaser by means of voice message. After confirmation of the purchase via the appropriate confirmation icon on the purchaser interface or the purchaser's voice message the operator-specialist unlocks the payment modules giving access to usage of cash or credit cards acceptors of the payment module.

After the payment by means of available for the purchaser payment acceptor the operator-specialist unlocks the door 9 of the container with appropriate number and the receipt and change dispensing module. The purchaser opens the door 9, takes the goods under control of the operator-specialist using the monitor camera 1 located on the doors 9 of the containers of the module with cells for goods. Dispensing of change and receipt is also carried out under control of the operator-specialist using the monitor camera of tray 13 similarly to the process performed during the purchase of goods of standard range.

During selling of the goods ordered in advance, for example, using the cells of medicines of the goods module of the vending machine, the purchaser is informed about limited time of the goods storage in the cell. In case during the specified time the purchaser fails to withdraw the goods (for example, medicine) from the cell, the goods are dispatched for storage to central office of which the purchaser is informed by means of the selected communication means (mail/phone). Afterwards the purchaser will have to withdraw the goods from the specified place.

It shall be noted that the vending machine and the method for selling of goods, in particular, medicines, specified in present description does not limit possible variations and modifications using the solutions of the related art within the scope and essence of the invention within the framework of the enclosed claims.

## Claims

1. A vending machine for remote selling of regulated goods containing a body (3) and following items installed in the body
communication unit providing possibility of establishing of communication with remote station of operators-specialists, herewith, the station of operators-specialists includes at least one computerized workstation of operator-specialist on regulated goods, software and hardware unit for data collection and control of operations of the vending machine for remote selling of regulated goods and database and data storage management server.
software and hardware unit of receipt and interpretation of control commands of the vending machine and related vending machine functional features and modules for acceptance, payment and dispensing of an order including
audio and video unit containing a data display device in form of a touch screen (4) with purchaser interface installed on a front panel of the body, video camera (5) located over the touch screen, loudspeakers (6a, 6b) and microphone (7) and
operator-specialist call button (8) equipped with a plate with a graphic pointer of designation in the form of Braille type located on the front panel and
controlled by the operator-specialist
module with cells for goods performed as containers with doors (9) on the front panel with the doors (9) monitor camera (17),
authorization document reading device in the form of scanner (12), at least one payment module with payment acceptor (10,11) on the front panel of the body,
goods dispensing module with tray (14) on the front panel of the body equipped with dispensing module equipped with shutter door (14b) giving out monitor camera,
change and receipt dispensing module with tray (13) on the front panel of the body equipped with monitor camera, and
goods return module with the tray (15) on the side panel of the body and documentation storage module with tray (16) on the side panel of the body equipped, accordingly, with controlled shutter doors (15b) and (16b) and monitor cameras of goods return and documentation dispensing, herewith
plates (10a, 11a, 13a, 14a, 15a) with graphic indicators of designation in form of Braille type are placed near the points of location of said payment acceptor (10,11), tray (13) of change and receipt dispensing module, tray (14) of goods dispensing module and tray (15) of goods return module.

2. The vending machine according to claim 1, wherein
the module with cells for goods is equipped with the device of keeping the temperature and humidity conditions.

3. The vending machine according to claim 1, wherein the authorization document reader is equipped with the container with monitoring camera for acceptance of prescription forms during selling of prescription medicines.

4. The vending machine according to claim 2 wherein
means of payment for goods is performed in the form of cash payment module and/or credit card payment module.

5. The vending machine according to claim 2, wherein
workstation of station of operators-specialists is the workstation of operator-pharmacist or operator-peddler.

6. A method for remote selling of regulated goods using the vending machine for remote selling of regulated goods according to claim 1, including
initialization of the vending machine from the standby mode by means of touching the touch screen by the purchaser with displaying of the purchaser interface on the screen or by means of pressing the call button on the front panel of the vending machine.
establishing of communication between the vending machine and the station of operators-specialists by the purchaser via wire or wireless communication unit by means of touching the icon "call" on the purchaser interface or pressing the call button on the front panel of the vending machine,
determining of available operator-specialist and upload of data on activated vending machine to computerized workstation of operator-specialist, displaying of operation interface of control of the vending machine functional devices and modules on the screen of workstation of operator-specialist and initiation of operation mode of the vending machine audio and video unit by the operator-specialist,
displaying a list of goods on the vending machine touch screen and selection by the purchaser of the goods for acquisition with submission of authorization documents for specific types of goods using the vending machine scanner with further displaying of a list of selected goods on the screen at the operator-specialist's workstation,
displaying of a purchase total on the vending machine touch screen or in the form of operator-specialist's voice message regarding the goods quantity and price,
confirmation of the selected goods by the purchaser by means of touching the selected goods confirmation icon on purchaser interface or by means of pressing the call button on the vending machine front panel.
unlocking of payment module of the vending machine by the operator-specialist,
payment by means of the selected payment modules acceptor with further unlocking of goods dispensing module and change and receipt dispensing module controlled by operator-specialist using the monitor cameras of the trays of goods dispensing module and change and receipt dispensing module,
putting the vending machine into standby mode by the operator-specialist or automatically upon expiration of time interval during which the vending machine is not used by the purchasers.

7. The method according to claim 6, wherein during selection of the goods the operator provides the purchaser access to the goods documentation by means of tray of the documentation storage module with its further return controlled by the operator-specialist using the monitor camera of the said tray.

8. The method according to claim 6 wherein,
return of the goods is performed by means of the goods return tray controlled by the operator-specialist using the monitor camera of the goods return tray.

9. A method for remote selling of regulated goods upon advance order using the vending machine for remote selling according to claim 1 including
receipt of message regarding the vending machine address and number and the time of the ordered goods delivery to the vending machine and the unique purchase code by the purchaser,
initialization of the vending machine from the standby mode by means of touching the touch screen by the purchaser with displaying of the purchaser interface on the screen or by means of pressing the call button on the front panel of the vending machine.
establishing of communication between the vending machine and the station of operators-specialists by the purchaser via wire or wireless communication unit by means of touching the icon "call" on the purchaser interface or pressing the call button on the front panel of the vending machine,
entering of the unique code via the purchaser interface on the vending machine screen or entering of the unique code by operator-specialist on basis of the purchaser's voice message sent via the microphone of the vending machine audio and video unit,
displaying of the purchase total on the vending machine touch screen or in the form of operator-specialist's voice message regarding the goods quantity and price,
confirmation of the order by the purchaser by means of touching the order confirmation icon on purchaser interface or by means of pressing the call button on the vending machine front panel.
reporting of the container number of the cells for goods module by the operator-specialist via the purchaser interface or by means of voice message
payment by the purchaser for the order by means of the selected payment modules acceptor with further unlocking of the container door of the module with cells for goods and change and receipt dispensing module controlled by operator-specialist using the monitor camera of the tray of change and receipt dispensing module,
getting of the medicine from the appropriate container controlled by the operator-specialist using the monitor camera 17 of doors 9 of the said containers,
putting the vending machine into standby mode by the operator-specialist or automatically upon expiration of time interval during which the vending machine is not used by the purchasers.
